# EUROPEAN PATENT APPLICATION

(11) **EP 2 561 836 A2**
(43) Date of publication of application: **27.02.2013**
(21) Application number: 11772194.4
(22) Date of filing: 19.04.2011
(51) Int. Cl.: A61F 2/82, A61L 27/28, A61L 27/54, A61L 27/34

(54) **VASCULAR STENT HAVING A DUAL COATING STRUCTURE**

(30) Priority: 21.04.2010 KR 20100036844
(71) Applicant: Bioalpha Inc., Seoul 135-090 (KR)
(72) Inventor: KIM, Sang Ho, Seongnam-si Gyeonggi-do 463-839 (KR); PARK, Jong Chae, Osan-si Gyeonggi-do 447-743 (KR); KIM, Eun Jin, Seongnam-si Gyeonggi-do 463-761 (KR); SHIN, Il Gyun, Anyang-si Gyeonggi-do 431-060 (KR); KIM, Dong Gon, Yeosu-si Jeollanam-do 555-808 (KR); KIM, Han Gi, Anyang-si Gyeonggi-do 430-825 (KR)
(74) Representative: Johnstone, Helen Margaret
(86) International application number: PCT/KR2011/002774
(87) International publication number: WO 2011/132906

(57) **Abstract**

Disclosed is a vascular stent which is inserted inside a blood vessel. The disclosed vascular stent comprises: a first coating film comprising a restenosis inhibiting drug provided on the outside surface of the stent strut; and a second coating film comprising an internal-capsule cellularization promoting drug provided on the inside surface of the stent strut. In this way, restenosis and thrombosis can be prevented from occurring inside the stent.

## Description

### TECHNICAL FIELD

The present invention relates to a medical stent, and more particularly, to a blood vessel stent having a dual coating structure for preventing in-stent restenosis and thrombosis.

### BACKGROUND ART

Generally, a percutanous coronary intervention (PCI) is a surgical treatment for inserting a balloon catheter into an artery in the wrist or the leg, moving the balloon catheter to a coronary artery, and broadening a blocked portion of the coronary artery by inflating the balloon in the case of a cardiovascular disorder based on myocardial infarction, angina, stenosis of the coronary artery, etc., and is generally accepted as an effective treatment for cardiovascular disorders. Such PCI may only involve expanding vessel wall of an artery simply by using a balloon catheter. However, PCI is generally performed to insert and install a stent formed of a thin metal net into a blood vessel to continuously support expanded vessel wall.

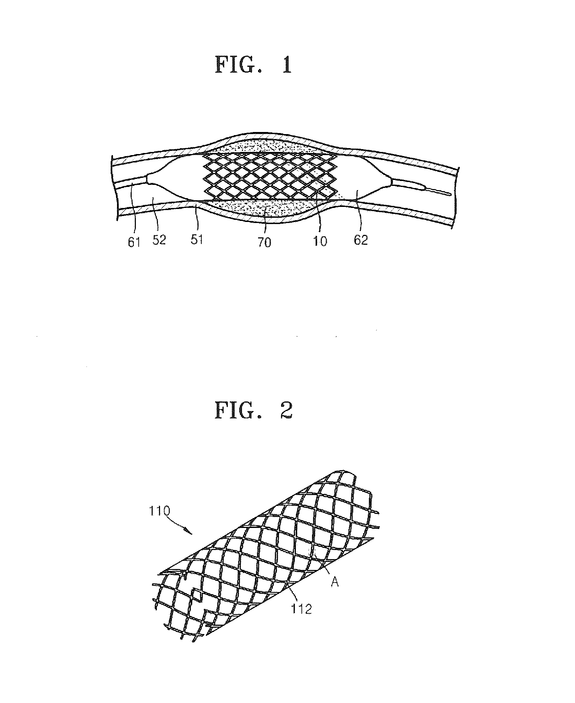

FIG. 1 shows that a PCI is performed by using a bare metal stent 10.

Referring to FIG. 1, a method of performing a PCI by using the bare metal stent 10 will be described. First, the bare metal stent 10 selected in consideration of a length of a plaque 70 stenosed to a vessel wall 51 and a diameter of a blood vessel is attached to a balloon catheter 61, and the balloon catheter 61 is pushed into an artery to reach a location of the stenosed plaque 70 via the interior 52 of the blood vessel. Next, after the balloon catheter 61 reaches the exact location of the plaque 70, a balloon 62 is inflated, and the bare metal stent 10 mounted on the balloon 62 is plastic-deformed and maintains an expanded state. As a result, the vessel wall 51 to which the plaque 70 is stenosed is expanded. Next, when the balloon 62 is contracted and the balloon catheter 61 is removed, only the bare metal stent 10 remains in the blood vessel and supports the expanded vessel wall 51, and thus the blood vessel is prevented from narrowing in the future.

However, in a case where a PCI is performed by using the bare metal stent 10, tissue cells of a vessel wall pushed and pressed by the bare metal stent 10 experience barotrauma and rapid proliferation of smooth muscle cells. When the proliferated smooth muscle cells excessively cover the bare metal stent 10, in-stent restenosis (re-blockage of the vessel wall 51) may occur. It is known in the art that such in-stent restenosis is mainly related to formation of an extracellular matrix due to rapid proliferation of smooth muscle cells moved from the middle layer of the vessel wall 51, that is, neointimal hyperplasia.

To resolve such in-stent restenosis problem, a surgery using a drug eluting stent for maintaining an expanded vessel wall and eluting a drug for suppressing proliferation of cells has been recently developed and is being used. The surgery using a drug eluting stent may reduce in-stent restenosis by suppressing proliferation of neointimal layers more effectively by suppressing proliferation and movement of smooth muscle cells. However, for arterial healing, a surgery using a drug eluting stent may induce more serious disorders than a surgery using a bare metal stent. Furthermore, since endothelialization occurs slowly, a drug eluting stent induces in-stent thrombosis more frequently than a bare metal stent.

### TECHINCAL PROBLEM

The present invention provides a blood vessel stent having a dual coating structure for preventing in-stent restenosis and thrombosis.

### ADVANTAGEOUS EFFECTS

According to the present invention, in-stent restenosis and thrombosis may be prevented by coating an outer surface of a stent strut with a drug for preventing restenosis and coating an inner surface of the stent strut with an endothelialization accelerating drug.

### DESCRIPTION OF THE DRAWINGS

The above and other features and advantages of the present invention will become more apparent by describing in detail exemplary embodiments thereof with reference to the attached drawings in which:
FIG. 1 shows that a PCI is performed by using a bare metal stent;
FIG. 2 is a perspective view of a blood vessel stent according to an embodiment of the present invention;
FIG. 3 is a partially exploded perspective view showing portion A of FIG. 2 in closer detail;
FIG. 4 is a sectional view taken along line IV-IV' of FIG. 3;
FIG. 5 is a sectional view taken along line V-V' of FIG. 3;
FIG. 6 is a partially exploded perspective view of the blood vessel stent according to an embodiment of the present invention that is inserted into a blood vessel;
FIG. 7 is a sectional view of a blood vessel stent according to another embodiment of the present invention;
FIG. 8 is a section view of a modification of the blood vessel stent shown in FIG. 7;
FIG. 9 is a sectional view of a blood vessel stent according to another embodiment of the present invention;
FIG. 10 is a sectional view of a blood vessel stent according to another embodiment of the present invention; and
FIG. 11 is a sectional view of a modification of the blood vessel stent shown in FIG. 10.

### BEST MODE

Hereinafter, the present invention will be described in detail by explaining preferred embodiments of the invention with reference to the attached drawings. Like reference numerals in the drawings denote like elements. In the drawings, the thicknesses of layers and regions are exaggerated for clarity.

As used herein, the term "and/or" includes any and all combinations of one or more of the associated listed items. Expressions such as "at least one of," when preceding a list of elements, modify the entire list of elements and do not modify the individual elements of the list.

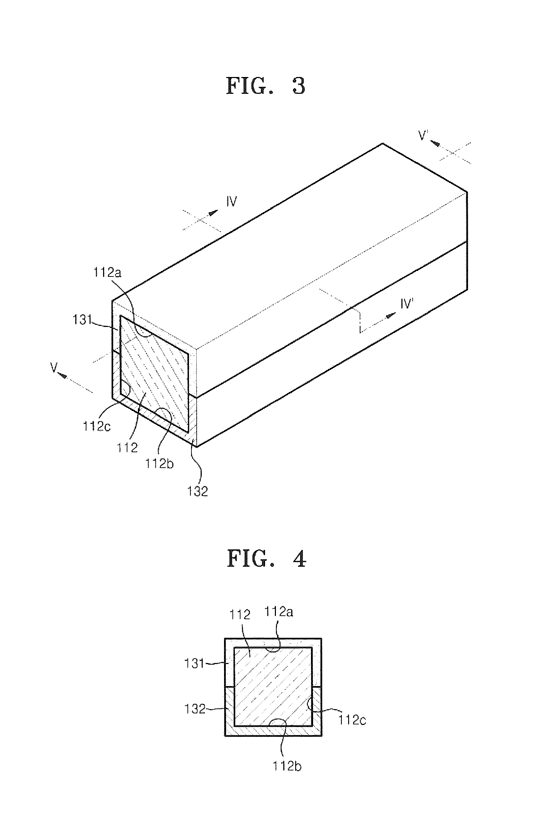

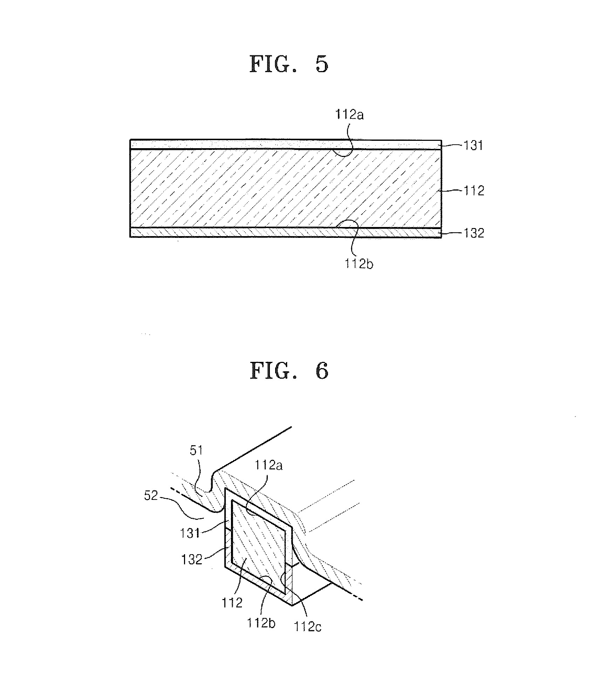

FIG. 2 is a perspective view of a blood vessel stent 110 according to an embodiment of the present invention, and FIG. 3 is a partially exploded perspective view showing portion A of FIG. 2 in closer detail. FIG. 4 is a sectional view taken along line IV-IV' of FIG. 3, and FIG. 5 is a sectional view taken along line V-V' of FIG. 3.

Referring to FIGS. 2 through 5, the blood vessel stent 110 includes a stent strut 112, a first coating layer 131 arranged on an outer surface 112a of the stent strut 112, and a second coating layer 132 arranged on an inner surface 112b of the stent strut 112. In detail, the stent strut 112 is a supporting unit of the blood vessel stent 110, is formed to have a mesh structure, and has a long and narrow cylindrical shape. Here, the stent strut 112 may have any of various mesh structure. The stent strut 112 is generally formed of a metal, such as stainless steel. However, the present invention is not limited thereto, and the stent strut 112 may be formed of any of various materials. The stent strut 112 may be manufactured by laser-processing a predetermined metal tube or using any of various other methods, such as laser-welding wire-like members, such as metal wires, or weaving a plurality of metal wires.

The stent strut 112 includes the outer surface 112a, the inner surface 112b, and lateral surfaces 112c. Here, the outer surface 112a of the stent strut 112 refers to a surface of the stent strut 112 which contacts a vessel wall (51 of FIG. 1) when the blood vessel stent 110 is inserted into a blood vessel, whereas the inner surface 112b of the stent strut 112 refers to a surface of the stent strut 112 contacting the interior of the blood vessel (52 of FIG. 1), in which blood flows, when the blood vessel stent 110 is inserted into the blood vessel. Furthermore, the lateral surfaces 112c of the stent strut 112 refer to two opposite surfaces of the stent strut 112 interconnecting the outer surface 112a and the inner surface 112b. FIGS. 3 through 6 show cases in which the stent strut 112 has a square cross-section or a rectangular cross-section. However, the stent strut 112 may have a cross-section having any of various other quadrangular shapes or any of various polygonal shapes other than quadrangular shapes.

The first coating layer 131 is arranged on the outer surface 112a of the stent strut 112. Here, the first coating layer 131 contains a drug for preventing restenosis. Furthermore, the first coating layer 131 may also contain a predetermined polymer together with the drug for preventing restenosis. Here, the polymer may be at least one of a biodegradable polymer and a biocompatible polymer. The first coating layer 131 may be formed by applying a solution, in which the drug for preventing restenosis, a biodegradable polymer, and/or a biocompatible polymer are mixed, onto the outer surface 112a of the stent strut 112 and drying the applied solution. Here, the solution may be applied via dipping, spraying, or any of various other methods. Here, the first coating layer 131 may extend from the outer surface 112a of the stent strut 112 to a portion of the lateral surfaces 112c of the stent strut 112. The first coating layer 131 arranged on the outer surface 112a of the stent strut 112 contacts a vessel wall (51 of FIG. 1; e.g., an arterial wall) and elutes a drug for preventing restenosis when the blood vessel stent 110 is inserted into a blood vessel.

The second coating layer 132 is arranged on the inner surface 112b of the stent strut 112. Here, the second coating layer 132 contains an endothelialization accelerating drug. Furthermore, the second coating layer 132 may further include a biodegradable polymer or a biocompatible polymer, like the first coating layer 131. The second coating layer 132 may be formed by applying a solution, in which the endothelialization accelerating drug, a biodegradable polymer, and/or a biocompatible polymer are mixed, onto the inner surface 112b of the stent strut 112 and drying the applied solution. Here, the second coating layer 132 may extend from the inner surface 112b of the stent strut 112 to a portion of the lateral surfaces 112c of the stent strut 112. When the blood vessel stent 110 is inserted into a blood vessel, the second coating layer 132 arranged on the inner surface 112b of the stent strut 112 contacts the interior of the blood vessel (52 of FIG. 1; more particularly, blood flowing inside the blood vessel) and elutes the endothelialization accelerating drug. As shown in FIG. 4, the two opposite ends of the first coating layer 131 and the two opposite ends of the second coating layer 132 may contact each other on the lateral surfaces 112c. Here, the boundaries between the first coating layer 131 and the second coating layer 132 may vary on the lateral surfaces 112c of the stent strut 112 according to design conditions of the blood vessel stent 110.

FIG. 6 is a partially exploded perspective view of the blood vessel stent 110 according to an embodiment of the present invention that is inserted into a blood vessel. Referring to FIG. 6, the first coating layer 131, which is arranged on the outer surface 112a of the stent strut 112 and contains the drug for preventing restenosis, contacts a vessel wall (51; e.g., an arterial wall) to which a plaque is stenosed. The second coating layer 132, which is arranged on the inner surface 112b of the stent strut 112 and contains the endothelialization accelerating drug, contacts blood flowing in the interior 52 of the blood vessel. While the blood vessel stent 110 according to an embodiment of the present invention is inserted into the blood vessel, the blood vessel stent 110 expands the vessel wall 51 and supports the expanded vessel wall 51, the first coating layer 131 contacting the vessel wall 51 elutes the drug for preventing restenosis, and the second coating layer 132 contacting blood in the interior 52 of the blood vessel elutes an endothelialization accelerating drug.

As described above, when the first coating layer 131, which is arranged on the outer surface 112a of the stent strut 112 and contacts the vessel wall 51, elutes the drug for preventing restenosis, proliferation and movement of cells, such as smooth muscle cells, may be suppressed, and thus the possibility of in-stent restenosis may be reduced. When endothelialization slowly occurs at the vessel wall 51, the possibility of in-stent thrombosis increases. However, when the second coating layer 132, which is arranged on the inner surface 112b of the stent strut 112, elutes the endothelialization accelerating drug, endothelialization rapidly occurs at the vessel wall 51, and thus in-stent thrombosis may be prevented.

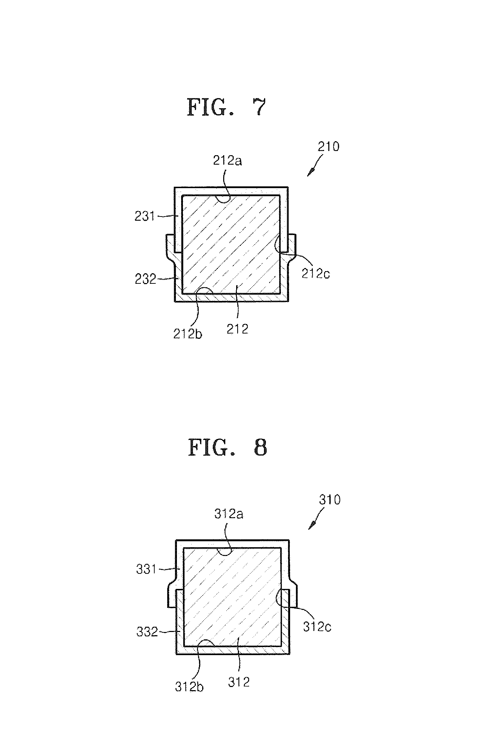

FIG. 7 is a sectional view of a blood vessel stent 210 according to another embodiment of the present invention. Hereinafter, only descriptions of the difference between the previous embodiment and the present embodiment are given. Referring to FIG. 7, a first coating layer 231 containing a drug for preventing restenosis is arranged on an outer surface 212a of a stent strut 212, whereas a second coating layer 232 containing an endothelialization accelerating drug is arranged on an inner surface 212b of the stent strut 212. Furthermore, two opposite ends of each of the first coating layer 231 and the second coating layer 232 extend onto lateral surfaces 212c of the stent strut 212, wherein the two opposite ends of the first coating layer 231 overlap with the two opposite ends of the second coating layer 232. In detail, the two opposite ends of the second coating layer 232 cover the two opposite ends of the first coating layer 231 on the lateral surfaces 212c. Locations of the two opposite ends of the first coating layer 231 and the second coating layer 232 may vary according to design conditions of the blood vessel stent 210. As described above, the first coating layer 231 and the second coating layer 232 may further include a biodegradable polymer or a biocompatible polymer.

FIG. 8 is a section view of a modification 310 of the blood vessel stent 210 shown in FIG. 7. Referring to FIG. 8, a first coating layer 331 containing a drug for preventing restenosis is arranged on an outer surface 312a of a stent strut 312, whereas a second coating layer 332 containing an endothelialization accelerating drug is arranged on an inner surface 312b of the stent strut 312. Furthermore, two opposite ends of each of the first coating layer 331 and the second coating layer 332 extend onto lateral surfaces 312c of the stent strut 312, wherein the two opposite ends of the first coating layer 331 cover the two opposite ends of the second coating layer 332 on the lateral surfaces 312c.

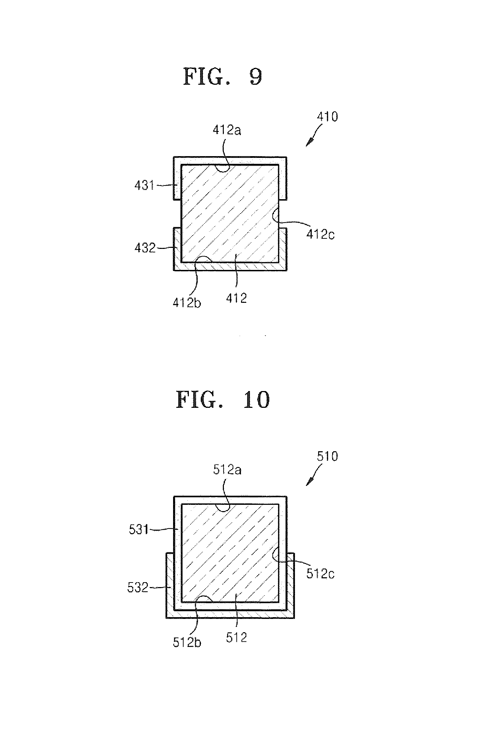

FIG. 9 is a sectional view of a blood vessel stent 410 according to another embodiment of the present invention. Referring to FIG. 9, a first coating layer 431 containing a drug for preventing restenosis is arranged on an outer surface 412a of a stent strut 412, whereas a second coating layer 432 containing an endothelialization accelerating drug is arranged on an inner surface 412b of the stent strut 412. Furthermore, two opposite ends of each of the first coating layer 431 and the second coating layer 432 extend onto lateral surfaces 412c of the stent strut 412, wherein the two opposite ends of the first coating layer 331 are formed to be apart from the two opposite ends of the second coating layer 332 on the lateral surfaces 312c. Locations of the two opposite ends of the first coating layer 431 and the second coating layer 432 may vary according to design conditions of the blood vessel stent 410.

FIG. 10 is a sectional view of a blood vessel stent 510 according to another embodiment of the present invention. Referring to FIG. 10, a first coating layer 531 containing a drug for preventing restenosis is arranged to surround all the surfaces of a stent strut 512, that is, an outer surface 512a, an inner surface 512b, and lateral surfaces 512c. Furthermore, a second coating layer 532 containing an endothelialization accelerating drug is arranged on a portion of the first coating layer 531 on the inner surface 512b of the stent strut 512. The second coating layer 532 may extend from the inner surface 512b of the stent strut 512 to the lateral surfaces 512c of the stent strut 512, wherein locations of the two opposite ends of the second coating layer 532 may vary according to design conditions of the blood vessel stent 510. As described above, if the first coating layer 531 is arranged on all the surfaces of the stent strut 512 and the second coating layer 532 is arranged on the portion of the first coating layer 531 on the inner surface 512b of the stent strut 512, the drug for preventing restenosis may be eluted from the outer surface 512a of the stent strut 512, whereas the endothelialization accelerating drug may be eluted from the inner surface 512b of the stent strut 512.

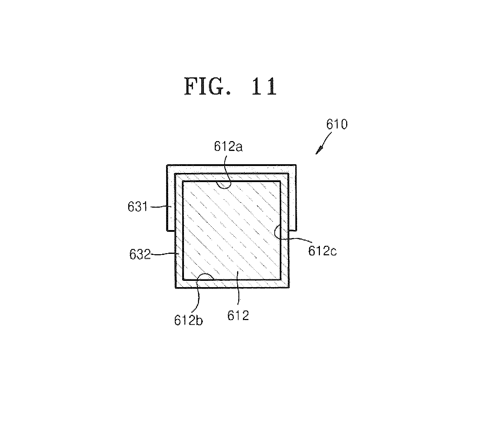

FIG. 11 is a sectional view of a modification 610 of the blood vessel stent 510 shown in FIG. 10. Referring to FIG. 11, a second coating layer 631 containing an endothelialization accelerating drug is arranged to surround all the surfaces of a stent strut 612, that is, an outer surface 612a, an inner surface 612b, and lateral surfaces 612c. Furthermore, a first coating layer 631 containing a drug for preventing restenosis is arranged on a portion of the second coating layer 632 on the outer surface 612a of the stent strut 612. The first coating layer 631 may extend from the outer surface 612a of the stent strut 612 to the lateral surfaces 612c of the stent strut 612, wherein locations of the two opposite ends of the first coating layer 631 may vary according to design conditions of the blood vessel stent 610. As described above, if the second coating layer 632 is arranged on all the surfaces of the stent strut 612 and the first coating layer 631 is arranged on the portion of the second coating layer 632 on the outer surface 612a of the stent strut 612, the drug for preventing restenosis may be eluted from the outer surface 612a of the stent strut 612, whereas the endothelialization accelerating drug may be eluted from the inner surface 612b of the stent strut 612

According to the present invention, in-stent restenosis and thrombosis may be prevented by coating an outer surface of a stent strut with a drug for preventing restenosis and coating an inner surface of the stent strut with an endothelialization accelerating drug.

Blood vessel stents according to embodiments of the present invention may be used on a heart artery for percutanous coronary intervention (PCI), for example. However, the present invention is not limited thereto, and blood vessel stents according to embodiments of the present invention may be applied to any of various other blood vessels.

While the present invention has been particularly shown and described with reference to exemplary embodiments thereof, it will be understood by those of ordinary skill in the art that various changes in form and details may be made therein without departing from the spirit and scope of the present invention as defined by the following claims.

### MODE OF THE INVENTION

According to an aspect of the present invention, there is provided a blood vessel stent to be inserted into a blood vessel, the blood vessel stent including a stent strut, which is formed to have a mesh structure and has a long and narrow cylindrical shape; a first coating layer, which is arranged on an outer surface of the stent strut which contacts a vessel wall and contains a drug for preventing restenosis; and a second coating layer, which is arranged on an inner surface of the stent strut which contacts the interior of a blood vessel and contains an endothelialization accelerating drug.

The first coating layer and the second coating layer may contain at least one of a biodegradable polymer and a biocompatible polymer.

The two opposite ends of the first coating layer may be arranged to contact the two opposite ends of the second coating layer on lateral surfaces of the stent strut, respectively.

The two opposite ends of the first coating layer may be arranged to be apart from the two opposite ends of the second coating layer on lateral surfaces of the stent strut.

The two opposite ends of the first coating layer may be arranged to overlap with the two opposite ends of the second coating layer on lateral surfaces of the stent strut, respectively.

The first coating layer may be arranged to cover all the surfaces of the stent strut, and the second coating layer may be located on the inner surface of the stent strut. Alternatively, the second coating layer may be arranged to cover all the surfaces of the stent strut, and the first coating layer may be located on the outer surface of the stent strut.

## Claims

1. A blood vessel stent to be inserted into a blood vessel, the blood vessel stent comprising:
a stent strut, which is formed to have a mesh structure and has a long and narrow cylindrical shape;
a first coating layer, which is arranged on an outer surface of the stent strut which contacts a vessel wall and contains a drug for preventing restenosis; and
a second coating layer, which is arranged on an inner surface of the stent strut which contacts the interior of a blood vessel and contains an endothelialization accelerating drug.

2. The blood vessel stent of claim 1, wherein the first coating layer and the second coating layer contain at least one of a biodegradable polymer and a biocompatible polymer.

3. The blood vessel stent of claim 1 or 2, wherein the two opposite ends of the first coating layer are arranged to contact the two opposite ends of the second coating layer on lateral surfaces of the stent strut, respectively.

4. The blood vessel stent of claim 1 or 2, wherein the two opposite ends of the first coating layer are arranged to be apart from the two opposite ends of the second coating layer on lateral surfaces of the stent strut.

5. The blood vessel stent of claim 1 or 2, wherein the two opposite ends of the first coating layer are arranged to overlap with the two opposite ends of the second coating layer on lateral surfaces of the stent strut, respectively.

6. The blood vessel stent of claim 1 or 2, wherein the first coating layer is arranged to cover all the surfaces of the stent strut, and
the second coating layer is located on the inner surface of the stent strut.

7. The blood vessel stent of claim 1 or 2, wherein the second coating layer is arranged to cover all the surfaces of the stent strut, and
the first coating layer is located on the outer surface of the stent strut.
